# EUROPEAN PATENT APPLICATION

(11) **EP 1 801 203 A1**
(43) Date of publication of application: **27.06.2007**
(21) Application number: 05781594.6
(22) Date of filing: 05.09.2005
(51) Int. Cl.: C12N 5/06

(54) **METHOD OF PRODUCING ADAPTATION MEDIUM FOR ASTROCYTE-LIKE CELLS**

(30) Priority: 06.09.2004 JP 2004259043
(71) Applicant: TANABE SEIYAKU CO., LTD., Osaka-shi, Osaka 541-8505 (JP)
(72) Inventor: Kondo, Yasushi, Kyoto-shi, Kyoto; 6128027 (JP); Suzuki, Yutaka, Sanda-shi, Hyogo 6691323 (JP); Okuno, Tsuyoshi, Sakai-shi, Osaka; 5918005 (JP)
(74) Representative: HOFFMANN EITLE
(86) International application number: PCT/JP2005/016245
(87) International publication number: WO 2006/028049

(57) **Abstract**

Disclosed are a method for preparing a conditioned medium of astrocyte-like cells derived from embryonic stem cells, **characterized in that** astrocyte-like cells, which are prepared by differentiation of embryonic stem cells, are cultured; the medium obtained by said method, the use of the conditioned medium for culturing neural cells as well as for inducing the differentiation of embryonic stem cells into neural cells.

## Description

### TECHNICAL FIELD

The present invention relates to a conditioned medium of astrocyte-like cells derived from embryonic stem cells. Specifically, the present invention relates to a conditioned medium of astrocyte-like cells derived from embryonic stem cells, a method for producing said conditioned medium, a method for culturing neurons using said conditioned medium of astrocyte-like cells derived from embryonic stem cells, neurons cultured by said method for culturing neurons, a method for inducing the differentiation of embryonic stem cells into neural cells using said conditioned medium of astrocyte-like cells derived from embryonic stem cells and neural cells prepared by inducing the differentiation of embryonic stem cells in the presence of the conditioned medium of astrocyte-like cells derived from embryonic stem cells.

### BACKGROUND ART

In order to culture neural cells, a synthetic culture medium which is a basal medium supplemented with some factors essential for neural survival (e.g. nerve growth factor and cytokines) or chemically-defined components, or a medium containing an astrocyte-conditioned medium have been used. Compared to a synthetic culture medium, a medium containing an astrocyte-conditioned medium can maintain neural cell cultures stably for a longer period.

It is also known that neural cells can efficiently be induced from embryonic stem cells by differentiating said cells in a medium containing an astrocyte-conditioned medium (see, non-patent documents 1 and 2).

As astrocyte-conditioned medium, a conditioned medium which is culture supernatant prepared by culturing primary astrocytes obtained from a neural tissue of an animal (e.g. rat, mouse, calf, horse, pig, monkey, rabbit or chicken) in nutrient medium has been used (see, patent documents 1 and 2).

However, the use of primary astrocyte may cause some problems. That is, availability of living (neural) tissues as the source for astrocytes is limited. It takes much time and efforts to obtain astrocytes from the living tissues. Additionally, it is very difficult to maintain a stable culture of primary cells in a culture vessel, and subculturing of them is limited within a few passages. Thus, in order to prepare a large amount of the astrocyte-conditioned medium according to the above described conventional method, huge efforts and time to repeat the process for the preparation as well as to acquire an enough amount of the living tissues have been required.

In addition, the properties of primary-cultured astrocytes vary depending on the maturation stage of the living body or the region of the living tissue from which the astrocyte is derived. Furthermore, contamination of cells other than the desired astrocytes is inevitable when they are obtained from the living body. Thus, it has been difficult to make a stable preparation of an astrocyte-conditioned medium having substantially uniform quality.

A lot of mediums for maintaining a neural culture, including serum-free mediums supplemented with various cytokines and/or growth factors, have been reported (see non-patent documents 3 and 4). In general, however, culture systems using the medium disclosed in those documents cannot maintain neural culture stably for a long time. If one of such mediums is effective to maintain the neural survival, it often promotes an additional glial proliferation which causes mixed culture of neurons and glia cells. Therefore, such culture system is not suitable for preparing homogeneous neuron culture which is used in, for example, examining a pharmacological effect of test compounds on neurons.

From a long time ago, it has been known that a conditioned medium of primary-cultured astrocytes can be used for culturing neurons. However, in terms of long-term culture of neurons, sole application of the conditioned medium of primary-cultured astrocytes has been revealed to be less effective than that had been reported. It is reported that a suitable medium for stable long-term culture of neurons can be prepared only by adding various additives (factors) into a conditioned medium of primary-cultured astrocyte (see patent documents 1 and 2).
[Patent document 1] Japanese patent application Laid Open No. JP-A-9-289891
[Patent document 2] Japanese patent application Laid Open No. JP-A-9-322765
[Non-patent document 1] Takashi Nakayama et al., Neuroreport. 2004 Mar 1;15(3):487-91. "Efficient production of neural stem cells and neurons from embryonic stem cells" [Non-patent document 2] Takashi Nakayama et al., Neurosci Res. 2003 Jun;46(2):241-9. "Astrocyte-derived factors instruct differentiation of embryonic stem cell into neurons"
[Non-patent document 3] Bottenstein JE et al., Proc Natl Acad Sci U S A. 1979 Jan;76(1):514-7. "Growth of a rat neuroblastoma cell line in serum-free supplemented medium"
[Non-patent document 4] Brewer GJ et al., Brain Res. 1989 Aug 7;494(1):65-74. "Survival and growth of hippocampal neurons in defined medium at low density: advantages, technique or low oxgen"

### DISCLOSURE OF INVENTION

### PROBLEMS TO BE SOLVED BY THE INVENTION

An object of the present invention is to provide a method for preparing a conditioned medium of astrocyte-like cells derived from embryonic stem cells. The method is effective to achieve at least one of the followings; stably supplying substantially uniform conditioned medium of astrocyte-like cells; supplying a large amount of conditioned medium of astrocyte-like cells by a simple procedure; supplying a conditioned medium of astrocyte-like cells containing substantially no cell components derived from an animal other than the targeted animal (e.g. supplying a conditioned medium of human astrocyte-like cells containing substantially no non-human cell component); supplying a conditioned medium of astrocyte-like cells which can be used to differentiate embryonic stem cells into neural cells efficiently; supplying a conditioned medium of astrocyte-like cells which can be used to maintain stable and healthy culture of isolated neurons for a long time and the like.

A further object of the present invention is to provide a conditioned medium of astrocyte-like cells prepared by the above mentioned method, which is useful for at least one of the followings: inducing differentiation of embryonic stem cells into neural cells efficiently; maintaining a stable and healthy neural culture for a long time and the like.

A further object of the present invention is to provide a method for culturing neurons, which can achieve at least one of the followings: maintaining a stable and healthy neural culture for a long time; culturing neurons derived from a targeted animal under the condition where components derived from an animal other than the targeted animal is not substantially present, and the like.

A further object of the present invention is to provide a method for differentiating embryonic stem cells into neural cells, which can achieve at least one of the followings: inducing the differentiation of embryonic stem cells into neural cells efficiently; inducing the differentiation of embryonic stem cells derived form a targeted animal into neural cells under the condition where component derived from an animal other than the targeted animal is not substantially present, and the like.

Furthermore, an object of the present invention is to provide neural cells which is induced from embryonic stem cells. By using said neural cells, at least one of the followings can be achieved: conducting cell transplantation treatment with substantially no contamination of a component originating from an animal other than the targeted animal (e.g. human); providing cells with high compatibility with a living body of the targeted animal (e.g. human), and the like.
Further objects of the present invention are apparent from the specification and conventional technology.

### MEANS TO SOLVE THE PROBLEMS

The present invention is made in light of the objects as mentioned above and relates to the followings:
[1] a method for preparing a conditioned medium of astrocyte-like cells derived from embryonic stem cells, characterized in that astrocyte-like cells, which are prepared by differentiation of embryonic stem cells, are cultured;
[2] The method of [1], wherein said embryonic stem cells are mammalian embryonic stem cells;
[3] The method of [2], wherein said mammal is a primate;
[4] The method of [3], wherein said primate is human;
[5] The method of any one of claims [1]-[4], comprising the steps of:
   (A) preparing astrocyte-like cells from embryonic stem cells, and
   (B) culturing the astrocyte-like cells prepared by said step (A) to give culture supernatant;
[6] The method of [5], in step (A) of the method, the embryonic stem cells are differentiated by using an conditioned medium of astrocytes, a preliminarily prepared conditioned medium of astrocyte-like cells, or a preliminarily prepared medium which is functionally-equivalent to said conditioned medium of astrocytes or conditioned medium of astrocyte-like cells;
[7] A conditioned medium of astrocyte-like cells derived from embryonic stem cells, which is prepared by the method of any one of [1]-[6];
[8] A method for culturing neurons, characterized in that the neurons are cultured in the presence of the conditioned medium of astrocyte-like cells derived from embryonic stem cells according to [7];
[9] Neurons which are cultured by the method according to [8];
[10] A method for inducing differentiation of embryonic stem cells into neural cells, characterized in that the embryonic cells are differentiated into neural cells in the presence of the conditioned medium of astrocyte-like cells derived from embryonic stem cells according to [7] and
[11] Neural cells which are obtained by differentiating embryonic stem cells using the conditioned medium of astrocyte-like cells derived from embryonic stem cells according to [7] .

### EFFECTS OF THE INVENTION

The method for preparing a conditioned medium of astrocyte-like cells derived from embryonic stem cells of the present invention can readily and stably provide a large amount of conditioned medium of astrocyte-like cells with substantially uniform quality. In addition, the method of the present invention can provide a conditioned medium of astrocyte-like cells containing substantially no component derived from an animal other than the targeted animal, for example, a conditioned medium of human astrocyte-like cells containing substantially no non-human component.

Further, a beneficial property of the conditioned medium of astrocyte-like cells derived from embryonic stem cells of the present invention is that the quality is substantially uniform. In addition, the conditioned medium of astrocyte-like cells derived from embryonic stem cells of the present invention can induce differentiation of embryonic stem cells into neural cells efficiently and it can also maintain neurons stably and healthfully for a long time. Furthermore, by using the conditioned medium of astrocyte-like cells derived from embryonic stem cells of the present invention, neural cells can be prepared and maintained under a condition where any component derived from an animal other than the targeted animal is not substantially present. For example, the conditioned medium of astrocyte-like cells derived from embryonic stem cells of the present invention can be used for preparing human neural cell culture (e.g. neural stem cells, neurons, astrocytes, and the like) containing substantially no non-human cell components.

In addition, according to the method for culturing neurons of the present invention, neurons can stably and healthfully be cultured for a long time under the condition where any components derived from animals other than the targeted animal are not substantially present. For example, according to the method for culturing neurons of the present invention, human neural culture can be maintained under the condition where any non-human components are not substantially present.

Further, according to the method for differentiating embryonic stem cells into neural cells of the present invention, neural cell culture containing substantially no component derived from an animal other than the targeted animal can be prepared from the embryonic stem cells efficiently. Therefore, the neural cells prepared by the method for inducing differentiation of the present invention will be useful for drug development, for example, for preparing cell transplantation materials for the regenerative therapy, for preparing assay systems (e.g. toxicity assessment, evaluation of efficacy, and the like) for discovery of new drugs.

Furthermore, the neural cells of the present invention can be used for cell transplantation substantially free from co-transplanting of a component derived from an animal other than the targeted animal. In addition, the neural cells of the present invention show a high compatibility with a living body of the animal (e.g. human) to be treated. The neural cells of the present invention can be used for an assay system for the screening of new drug to develop a drug suitable for the animal (e.g. human) to be treated.

### BRIEF DESCRIPTION OF DRAWINGS

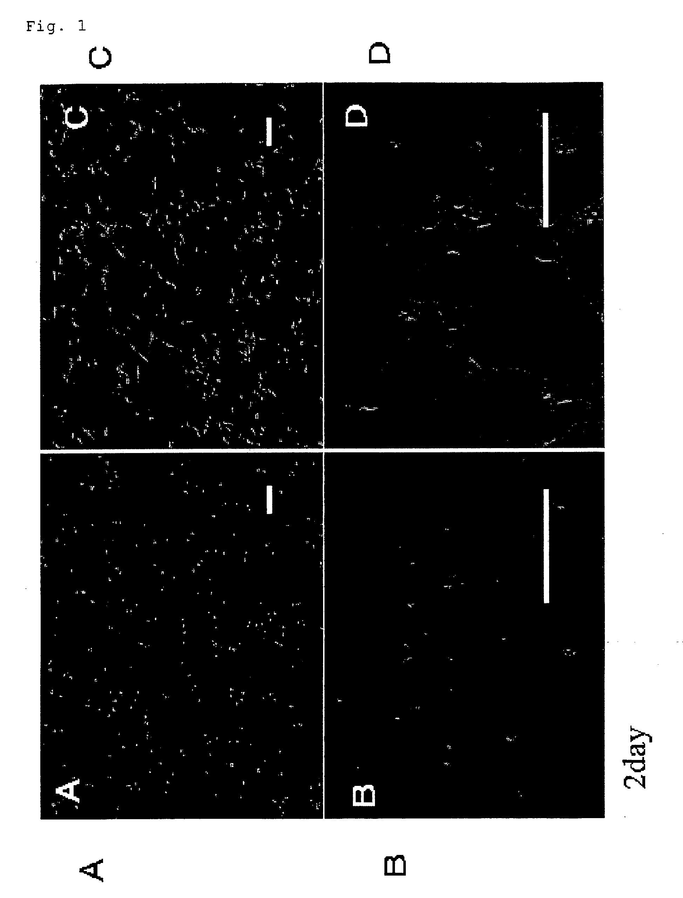

Figure 1 shows pictures of cell morphology prepared by culturing primary neurons derived from mouse fetal cortex in the conditioned medium of astrocyte-like cells derived from mouse embryonic stem cells or N2 medium (DMEM:F-12+N2 supplement) for 2 days. Panels A and B show the case of using N2 medium, and Panels C and D show the case of using the conditioned medium of astrocyte-like cells derived from mouse embryonic stem cells. In figures, bar scale indicates 50µm.

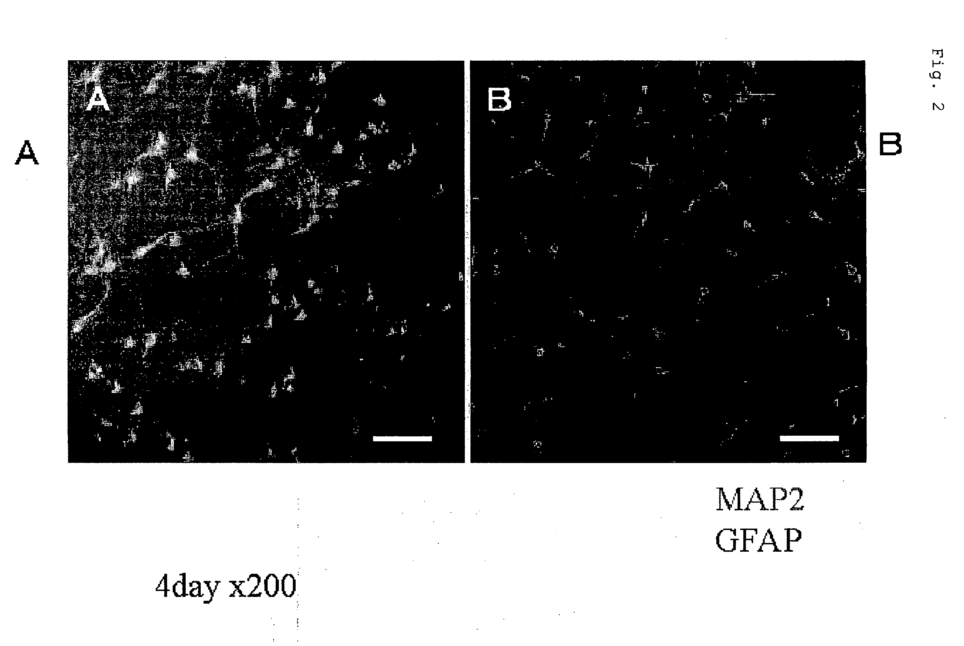

Figure 2 shows a detection of the expression of GFAP (glial fibrillary acidic protein) and MAP2 (microtubule-associated protein 2) respectively, by immunostaining. Panel A shows the case of using N2 medium, and Panel B shows the case of using the conditioned medium of astrocyte-like cells derived from mouse embryonic stem cells. The expression of MAP2 was detected by green fluorescence and the expression of GFAP was detected by red fluorescence. In figures, bar scale indicates 50µm.

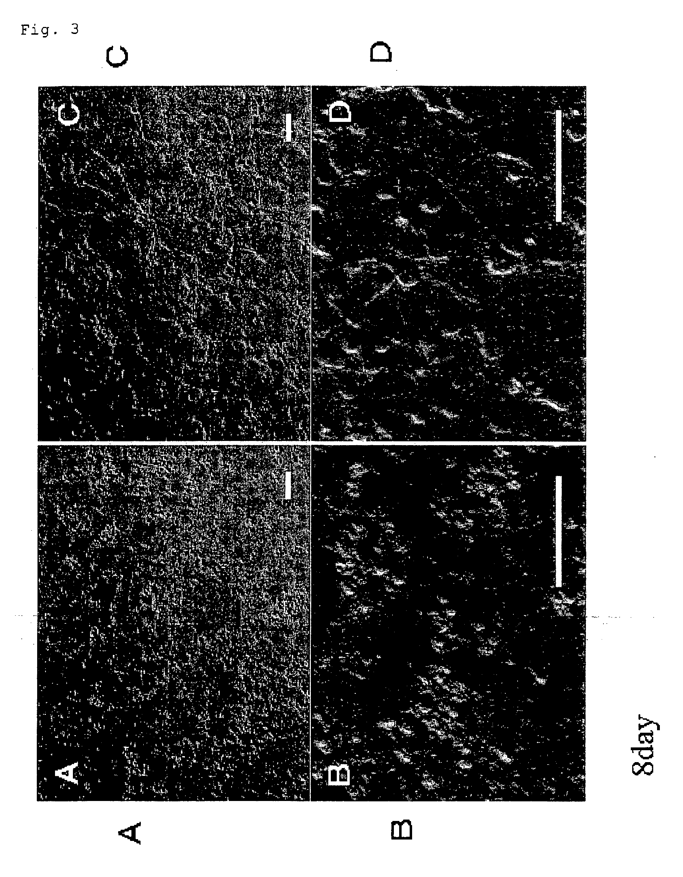

Figure 3 shows pictures of day 8 cells cultured in the conditioned medium of astrocyte-like cells derived from embryonic stem cells or Neurobasal^{™} medium with B-27 supplement. Panels A and B show the cells cultured in Neurobasal^{™} medium with B-27 supplement, and Panels C and D show the cells cultured in the conditioned medium of astrocyte-like cells derived from mouse embryonic stem cells. In figures, bar scale indicates 50µm.

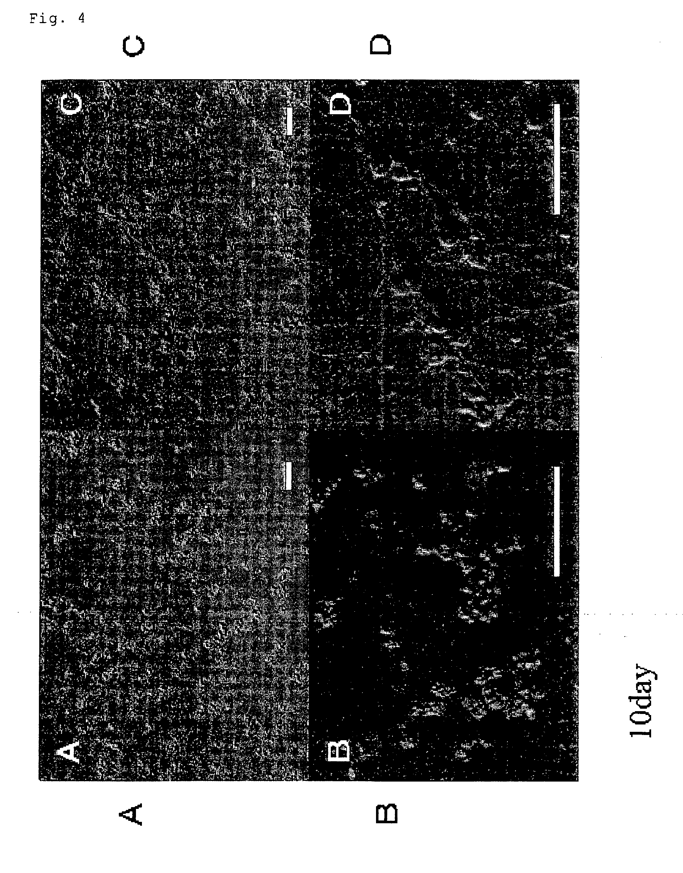

Figure 4 shows pictures of day 10 cells cultured in the conditioned medium of astrocyte-like cells derived from embryonic stem cells or Neurobasal^{™} medium with B-27 supplement. Panels A and B show the cells cultured in Neurobasal^{™} medium with B-27 supplement, and Panels C and D shows the cells cultured in the conditioned medium of astrocyte-like cells derived from mouse embryonic stem cells. In figures, bar scale indicates 50µm.

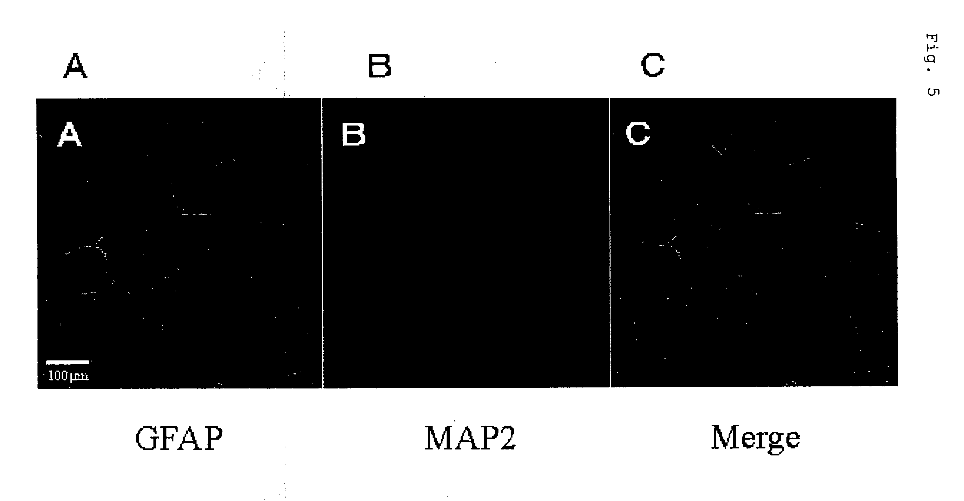

Figure 5 shows atrocyte-like cells prepared from monkey embryonic stem cells. This figure indicates immunostaining with anti-GFAP antibody or anti-MAP2 antibody; Panel A shows the expression of GFAP (green fluorescence), Panel B shows the expression of MAP2 (red fluorescence) and Panel C shows a merged image of Panels A and B. In figures, bar scale indicates 100µm.

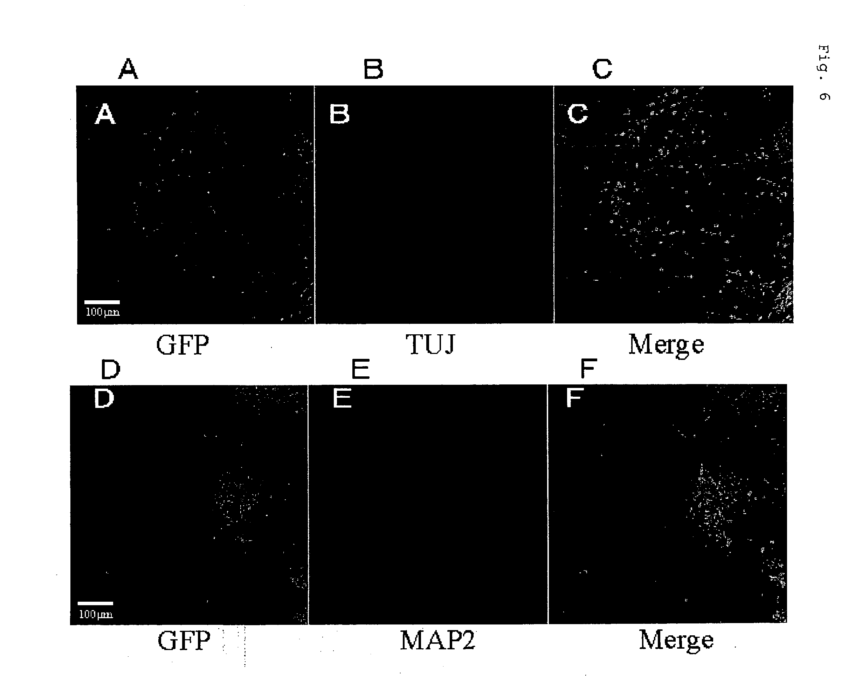

Figure 6 shows a picture of neurons (neural cells) which were differentiated from monkey embryonic stem cells using the conditioned medium of astrocyte-like cells derived from monkey embryonic stem cells. Panels A and D show the immunostained cells (green fluorescence indicates constitutive expression of hrGFP, and also the localization of monkey embryonic stem cells (CMK6-G4 cell line) which are used in the experiment and the cells differentiated from them), and Panels B and E show the expression of βIII tubulin (red fluorescence, corresponding to Panel A) and MAP2 (red fluorescence, corresponding to Panel D) respectively, and Panels C and F show the merged images of Panels A and B, and Panels D and E, respectively. In figures, bar scale indicates 100µm.

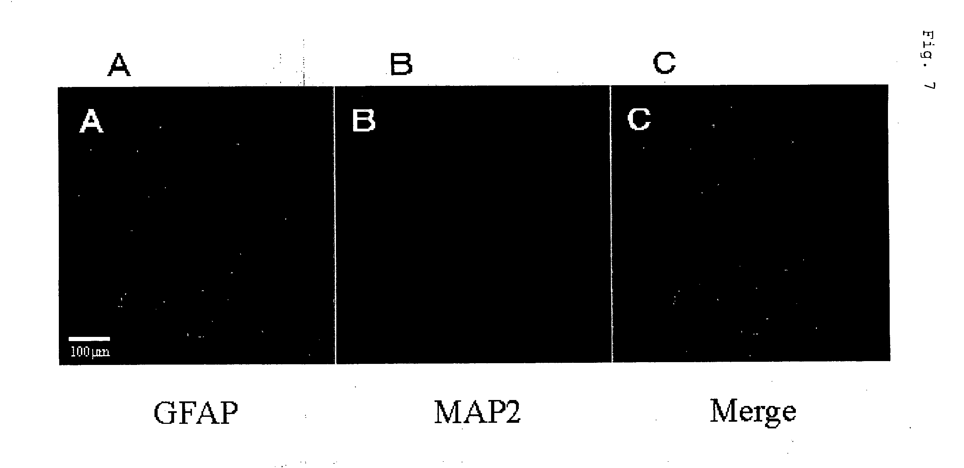

Figure 7 shows pictures of astrocyte-like cells prepared from human embryonic stem cells. The immunostaining with anti-GFAP antibody and anti-MAP2 antibody is shown in Panels; Panel A shows the expression of GFAP (green fluorescence), Panel B shows the expression of MAP 2 (red fluorescence), and Panel C shows a merged image of Panels A and B. The bar scale indicates 100µm.

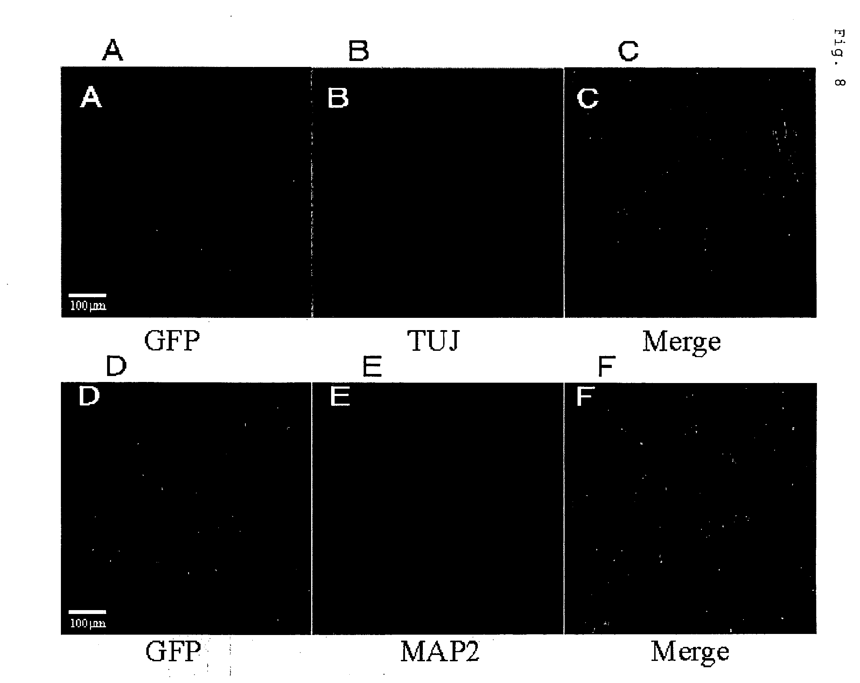

Figure 8 shows pictures of neurons (neural cells) which were differentiated from human embryonic stem cells using the conditioned medium of astrocyte-like cells derived from human embryonic stem cells. Panels A and D show pictures of immunostained cells (green fluorescence indicates constitutive expression of hrGFP, and also the localization of human embryonic stem cells (SA181hrG2 cell line) which are used in the experiment and the cells derived from them), and Panels B and E show the expression of βIII tubulin (red fluorescence, corresponding to Panel A) and MAP2 (red fluorescence, corresponding to Panel D) respectively, and Panels C and F show the merged images of Panels A and B, and Panels D and E, respectively. In figures, bar scale indicates 100µm.

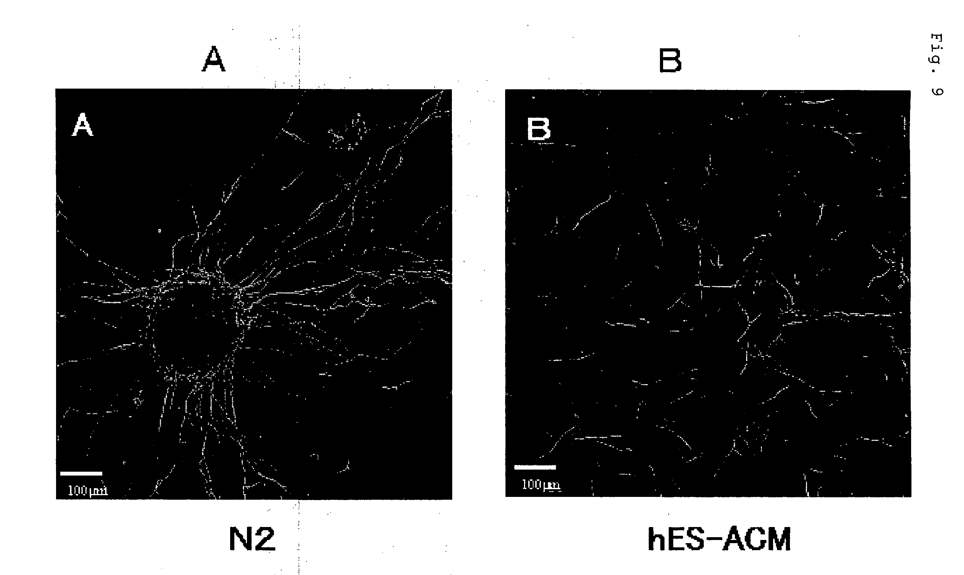

Figure 9 shows class III β tubulin-immunopositive cells detected by immunostaining in the cell population prepared by culturing human neural progenitor cells in the conditioned medium of astrocyte-like cells derived from human embryonic stem cells or N2 medium for 14 days. Panel A indicates the case using N2 medium (N2) and Panel B indicates the case using the conditioned medium of astrocyte-like cells derived from human embryonic stem cells (hES-ACM). While using N2 medium, areas as observed in Panel A are sparsely found in the culture vessel, using hES-ACM, neurons are seen all over the surfaces of the culture vessel, as observed in Panel B. In figures, bar scale indicates 100µm.

### BEST MODE FOR CARRYING OUT THE INVENTION

The present invention is based on the surprising evidence that a conditioned medium prepared by culturing astrocyte-like cells derived from embryonic stem cells can be used for neural cell culture or embryonic stem cells differentiation into neural cells, and that the ability of the medium to maintain neural cell culture or to induce neural differentiation is equal to or greater than that of the conditioned medium of primary-cultured astrocytes.

"Astrocyte-like cells" as used herein is defined as a cell population expressing Glial fibrillary acidic protein (GFAP) which is differentiated from embryonic stem cells. On the other hand, "primary-cultured astrocyte" refers to a cell population expressing GFAP which is obtained by dissociation of individual cells after treating a tissue removed from a living body with a cell dissociating agent such as trypsin. Therefore, various types of cells constituting the tissue are contaminated in such primary-cultured astrocytes.

Unless otherwise stated, "conditioned medium of astrocyte" as used herein refers to culture supernatant of the above mentioned primary-cultured astrocyte.

According to the present invention, a conditioned medium of astrocyte-like cells which is prepared by differentiating embryonic stem cells is described herein as "conditioned medium of astrocyte-like cells" or "conditioned medium of astrocyte-like cells derived from embryonic stem cells".

Sometimes, the above mentioned "conditioned medium of astrocyte", "conditioned medium of astrocyte-like cells" of the present invention and "conditioned medium of astrocyte-like cells derived from embryonic stem cells" of the present invention are generically referred to "conditioned medium" or "conditioned medium of the present invention".

In addition, the term "neural cells" as used herein includes neural stem cells, neurons, glia cells (e.g. astrocytes, oligodendrocytes), and the like. The glia cells refer to all cells supporting neurons.

One aspect of the present invention is to provide a method for preparing a conditioned medium of astrocyte derived from embryonic stem cells, characterized in culturing astrocyte-like cells prepared by differentiation of embryonic stem cells. According to the method of the present invention, a large amount of conditioned medium having a beneficial property in inducing differentiation of embryonic stem cells into neural cells efficiently, can be provided stably in a simple way. According to the method of the present invention, a large amount of conditioned medium having a beneficial property of maintaining neural culture healthfully can be provided stabely for a long time, in a simple way.

One of the features of the method of the present invention is the use of astrocyte-like cells prepared by differentiation of embryonic stem cells for preparing the conditioned medium. The ability of the conditioned medium prepared by the method of the present invention to induce the differentiation of embryonic stem cells into neural cells is similar to, or greater than, that of a conditioned medium prepared by using primary-cultured astrocytes. In addition, by employing the conditioned medium prepared by the present invention, the art can avoid the problems which may be caused by the use of primary-cultured astrocytes, i.e. variation in property or condition of the medium depending on the part or age of the living body from which the astrocytes are derived and inevitable contamination of cells other than astrocytes, and a conditioned medium of astrocyte-like cells derived from embryonic stem cells with substantially uniform quality can be prepared.

The astrocyte-like cells used in the method of the present invention are prepared by differentiating embryonic stem cells that have indefinite proliferative potential under a certain condition, and therefore, have superior mass productivity and quality stability. Thus, the method of the present invention allows to provide a large amount of conditioned medium stably, and is used more effectively in the manufacture of the conditioned medium compared to the method using primary-cultured astrocytes.

Further, according to the method of the present invention, a conditioned medium of astrocyte-like cells derived from embryonic stem cells containing substantially no cell component derived form an animal other than the targeted animal can be provided, because the method of the present invention does not use primary-cultured astrocytes derived from a living body. For example, according to the method of the present invention, a conditioned medium of astrocyte-like cells derived from embryonic stem cells containing substantially no non-human cell component. Therefore, for example, using a conditioned medium of astrocytes derived from human embryonic stem cells, human neural cells can be prepared from human embryonic stem cells in a process where non-human cell component is not substantially present. The method of the present invention can be applied for manufacturing regenerative medicine used for cell transplantation therapy and for developing new drug using primate cells, especially human cells. The method of the invention enables to provide substantially pure neural culture of primate, especially human as well as to culture the pure neural cells stably for long time.

The embryonic stem cells used in the method of the present invention may be derived from any animal depending on the purpose of use. Specifically, embryonic stem cells derived from a mammal are preferably used. More specifically, examples of the mammals may include mouse, rat, mink, hamster, pig, monkey (e.g. marmoset, rhesus macaque, cynomolgus, and the like) and human. According to the method of the present invention, in order to prepare a conditioned medium for preparing or maintaining neurons to be used in the cell transplantation therapy, the embryonic stem cells used in the method may preferably be the cells derived from the transplant recipient from the viewpoint of histocompatibility. That is, when the conditioned medium is prepared for the purpose of preparing or maintaining neural cells which are used for transplanting in a human patient, the embryonic stem cells derived from the recipient are preferably used in the method for preparing the conditioned medium.

In one embodiment of the method of the present invention, a method comprising the steps of:
(A) preparing astrocyte-like cells from embryonic stem cells, and
(B) culturing said astrocyte-like cells prepared by the process (A) to provide culture supernatant is provided.

More specifically, said process (A) may include, but is not limited to, the steps of:
1) differentiating embryonic stem cells into neural stem cells using the astrocyte-conditioned medium,
2) proliferating said neural stem cells, and
3) inducing selective differentiation of said proliferated neural stem cells into astrocyte-like cells.

In the above step 1), differentiation of embryonic stem cells to neural stem cells can be accomplished by, for example, culturing embryonic stem cells in an astrocyte-conditioned medium. Optionally, in the above step 1), a synthetic medium containing a basal medium supplemented with some factors such as growth factors and cytokines, or chemically-defined components may be mixed with said astrocyte-conditioned medium or a medium equivalent to said astrocyte-conditioned medium.

In the method of the present invention, once astrocyte-like cells derived from embryonic stem cells are established, the astrocyte-like cells-conditioned medium prepared by culturing said astrocyte-like cells derived from embryonic stem cells may be used instead of the astrocyte-conditioned medium. The astrocyte-like cells-conditioned medium of the present invention may continuously be prepared by employing thus obtained astrocyte-like cells-conditioned medium of the present invention. In a preferred embodiment of the present invention, the step 1) is replaced by the step 1'): 1') differentiating embryonic stem cells to neural stem cells using a conditioned medium of astrocyte-like cells derived from embryonic stem cells.

The differentiation in said steps 1) and 1') can be induced, for example, by culturing the cells in suspension culture.

More specifically, in said step 1) or 1'), whole undifferentiated colonies of embryonic stem cells are cultured in suspension to provide cell spheres (so-called "Neural stem sphere(s)"; NSS), wherein a number of neural stem cells are preferably localized on the surface of said NSS. "Neural stem cell" as used herein refers to a precursor cell which differentiates into astrocyte-like cells and can be identified as positive cells expressing neural stem cells' markers such as nestin and musashi as well as glial precursors' markers such as A2B5.

Any culture vessels having suitable shape and size for maintaining stable continuous suspension culture in the manner that the cell spheres (i.e. NSS) do not adhere to the bottom of the culture vessel may be used for this step. Examples of such culture vessels include a dish for suspension culture and petri dish for bacterial culture. Optionally, said culture vessel may be coated with 0.5 % by weight of agarose to prevent the adhesion of the cell spheres to the bottom.

The period for the suspension culture may be determined depending on the type of the embryonic stem cells. For example, the period for the suspension culture can be determined based on the appearance of neural stem cells which are astrocyte precursor cells on the surface of the NSS. Said neural stem cells can be detected, for example, based on the expression of neural stem cells' markers such as nestin and musashi or of glial precursors' marker such as A2B5. In addition, the efficacy of inducing differentiation into neural cells in suspension culture may be examined by detecting the expression of immature neural markers, such as βIII tubulin, using immunohistochemical techniques. Typical examples of the periods for culture are as follows; usually 1-10 days, preferably about 4 days (e.g. 3-5days) for mouse embryonic stem cells, usually 1-15 days, preferably about 7 days (e.g. 5-8 days) for primate (including monkey, human) embryonic stem cells.

Next step is the proliferation of the neural stem cells [step 2]. In said step 2), the neural stem cells prepared by above mentioned step 1) itself or NSS including said neural stem cells are cultured in an adherent culture system. Neural stem cells are proliferated by conducting said step 2). In addition, when NSS including neural stem cells are cultured in the manner as above, neural stem cells are migrated from the adhered NSS and proliferated around the NSS. Accordingly, a large amount of neural stem cells are proliferated by conducting said step 2) and thereby a large amount of astrocyte-like cells can be prepared in the following step 3).

Examples of mediums suitable for proliferating neural stem cells in adherent culture of the NSS in the above mentioned step 2) include DMEM: F-12(1:1) and Neurobasal^{™} Medium (Invitogen Corporation) which are supplemented with B27 supplement [Brewer, G.J., Focus, 16, 6-9 (1994); Brewer, G.J., J. Neurosci. Res., 35, 567-576 (1993)] [Invitrogen Corporation, Catalog No:17504-044] and proliferative/growth factors such as basic fibroblast growth factor (bFGF) and epidermal growth factor (EGF). Specifically, neural stem cells can be proliferated in an adherent culture system using Neurobasal Medium containing B27 supplement, bFGF, and optionally EGF, or DMEM:F-12 (1:1) containing N2 supplement, bFGF, and optionally EGF.

Preferably, the culture vessel used for the adherent culture of the neural stem cells is coated with Matrigel^{™} (made by BD Bioscience), poly-L-lysine, laminin or the like, so as to facilitate the adherence of NSS or the moved and proliferated neural stem cells on the vessel as well as to maintain the conditions of neural stem cells stably.

When using Matrigel^{™}, the coating of said culture vessels may be conducted according to the manufacture's instructions. When using the other extracellular substratum, the coating may be applied by a conventional method. For example, a solution containing the extracellular substratum may be applied to the vessel so that the bottom of the culture vessel is covered well and then the vessel is allowed to stand for a certain period or is incubated at 37°C.

In case of using said proliferative/growth factors such as bFGF and EGF, the concentration of the factors may appropriately be determined depending on the type of the embryonic stem cells or the like. In case of bFGF, the desirable concentration is 1-200ng/ml, preferably 20-40ng/ml. In case of EGF, desirable concentration is 10-50ng/ml, preferably 10-20ng/ml. Alternatively, in case of combined use of bFGF and EGF, they may be mixed to be 10-20ng/ml of bFGF and 10-20ng/ml of EGF.

The period for the above-described adherent culture may be determined depending on the type of animals from which the embryonic stem cells are derived, the degree of differentiation or the like.

In the above mentioned step 2), further proliferation of neural stem cells can be achieved by detaching the moved and proliferated neural stem cells from the culture vessel and dissociating the cells individually using a cell-dispersant, for example, an enzyme such as trypsin, dispase, collagenase, papain or the like, EDTA, or cell dissociation buffer [Gibco-Invitrogen], and then subculturing the dissociated cells in freshly prepared culture system. Specifically, for example, 0.05-0.35% by weight of trypsin may be used to detach the moved and differentiated neural stem cells and to dissociate the same individually. Thus dissociated cells may be subcultured and proliferated further.

In the step, undifferentiated embryonic stem cells located in the center of the cell population may optionally be eliminated from the culture to decrease the contamination of cells, such as undifferentiated embryonic stem cells, other than neural stem cells and increase the abundance ratio of the neural stem cells.

Next is the step of inducing differentiation of neural stem cells proliferated in step 2) selectively into astrocyte-like cells[step 3].

The condition for the selective induction of differentiation into astrocyte-like cells can be defined depending on the type of the starting embryonic stem cells. For example, it can be achieved by replacing the medium used in said step 2) with a medium containing no proliferative/growth factors such as bFGF and EGF.

Astrocyte-like cells derived from embryonic stem cells can be prepared as a population including the cells expressing GFAP by conducting the steps 1)-3) of the above mentioned step (A).

Next is the step of culturing astrocyte-like cells derived from embryonic stem cells, which is prepared by the step (A) and then, obtaining the culture supernatant[step (B)].

In the step (B), conditions for culturing astrocyte-like cells derived from embryonic stem cells are not limited as long as said astrocyte-like cells are able to survive and various factors can be released from said astrocyte-like cells. Using serum free medium is preferable when preventing a contamination of cell components derived from other animals is required for the process of manufacture. Specifically, for example, DMEM:F-12 medium containing N2 supplement (×1: 5µg/ml insulin, 10µg/ml transferrin, 63ng/ml progesterone, 16.11µg/ml putrescine and 2ng/ml selenite) is employed and cells are cultured at 37°C, about 5% CO₂.

So as to sufficiently release various factors produced by astrocyte-like cells into culture medium, the period for culture in the step (B) can be determined depending on the type of animals, the number of cells or the like. Usually, for example, the period may be about 1 day.

After culturing, the supernatant of the obtained culture is harvested to give conditioned medium. Specifically, the conditioned medium of the astrocyte-like cells can be obtained as a cell free culture supernatant by centrifuging the whole culture or a part of the culture supernatant and then, applying it to the filter having an appropriate pore size to exclude the cells.

When a whole culture is recovered and astrocyte-like cells are removed to give the conditioned medium, the conditioned medium of astrocyte-like cells derived from embryonic stem cells can be produced repeatedly by applying the medium and supplements used in step (B) to the astrocyte-like cells removed from the culture and repeating the series of steps under the same conditions. Alternatively, when a part of culture supernatant is recovered from the culture, the conditioned medium of astrocyte-like cells derived from embryonic stem cells can be produced repeatedly by applying the medium and supplements used in step (B) to the residue of the culture and repeating a series of the steps under the same conditions. The method of the present invention also covers the embodiments as above.

The astrocyte-like cells conditioned medium derived from embryonic stem cells prepared by the method of the present invention is effectively used for inducing differentiation of embryonic stem cells into neural cells. In addition, the conditioned medium of astrocyte-like cells derived from embryonic stem cells prepared by the method of the present invention can maintain a stable and healthy neural culture for a long time.

The conditioned medium of astrocyte-like cells derived from embryonic stem cells prepared by the method of the present invention can be evaluated by determining the ability to maintain neural culture and the ability to induce differentiation of embryonic stem cells into neural cells.

Said ability to maintain neural culture can be examined, for example, by culturing primary neurons obtained from a living body or neurons differentiated from embryonic stem cells, and then, analyzing the cultured neurons' conditions (e.g. survival and morphology) over time or at regular intervals. Said neural culture can be prepared in the condition described in, for example Neuroscience labomanual "The method for neuronal cell culture", editorial supervisor: Hachiro Nakagawa, editor: Hiroshi Hatanaka, publisher, Springer-Verlag Tokyo, page 347(35) (April 24, 1997).

In order to evaluate the ability to maintain neural culture, neurons may be cultured in the presence of the conditioned medium to be evaluated under the appropriate conditions. The morphology of the neurons is evaluated based on the presence or absence of cell body, dendrite, axon, growth cone and the like. In addition, for example, the expressions of markers including receptors of neurotransmitters, neurofilament, tyrosine hydroxylase, glutamic acid decarboxylase, choline acetyltransferase are examined by the detection using immunohistochemistry. If the expressions of those markers are detected and particular morphologies are observed, said conditioned medium can be evaluated as the one enabling to maintain healthy neurons.

Another aspect of the present invention relates to the conditioned medium of astrocyte-like cells derived from embryonic cells, which can be prepared according to the method of the present invention. The conditioned medium of astrocyte-like cells derived from embryonic cells of the present invention is obtained from the culture supernatant of astrocyte-like cells prepared by differentiating embryonic stem cells and therefore, may be more stable in quality compared to the conditioned medium of primary-cultured astrocytes. In addition, by using the conditioned medium of astrocyte-like cells derived from embryonic stem cells together with embryonic stem cells, neural cells can be prepared in the process substantially free from contamination of a component derived from an animal other than the targeted animal. Therefore, by using the conditioned medium of astrocyte-like cells derived from embryonic stem cells of the present invention, neural cell culture with still high quality containing substantially no component other than those derived from the targeted animal can be provided. Thus provided neural cell culture can be utilized for screening or cell toxicity evaluation in the drug development process. In addition, it can also be used for preparing highly compatible cells for transplantation used in the regenerative treatment.

In addition, by using the conditioned medium of astrocyte derived from embryonic stem cells of the present invention, embryonic stem cells can efficiently be differentiated into neural cells. It can also maintain a stable and healthy neural culture for a long time.

The conditioned medium of the astrocyte-like cells derived from embryonic stem cells of the present invention may contain additives including N2 supplement [insulin, transferrin, selenite, progesterone; see, for example, Bottenstein et al., Proc. Natl. Acad. Sci. USA., 76:514 (1979)], albumin, the above mentioned antioxidant or the like. The amount of said additives contained in the conditioned medium of astrocyte-like cells derived from embryonic stem cells of the present invention may be determined depending on the purpose of use, types of the cells to be cultured such as embryonic stem cells or neural cells and animal species from which the cells are obtained. The concentration of the components contained in the above mentioned N2 supplement may be, but are not limited to, 1-100µg/ml, preferably 3-20µg/ml of the final concentration for insulin, 1-100µg/ml, preferably 3-20µg/ml of the final concentration for transferrin, 1-100nM, preferably 3-50nM of the final concentration for selenite, 1-100nM, preferably 20nM of the final concentration for progesterone.

The conditioned medium of astrocyte-like cells derived from embryonic stem cells of the present invention may further contain some factors which are suitable for cell culture and allows to induce the desired differentiation depending on the purpose of use, types of cells to be cultured, animal species from which the cells are obtained, or the like.

In addition, said conditioned medium of astrocyte-like cells derived from embryonic stem cells of the present invention may comprise a conventional basal medium for culturing cells such as embryonic stem cells, neural stem cells or the like, in order to maintain the stable culture of cells such as neural cells for long time as well as to induce the differentiation efficiently. The ratio between said conditioned medium of astrocyte-like cells derived from embryonic stem cells and the basal medium may be determined depending on the purpose of use, types of cells to be cultured, animal species from which said cells are derived, or the like.

The conditioned medium of astrocyte-like cells derived from embryonic stem cells can be preserved in a frozen or chilled state. In order to store the conditioned medium of astrocyte-like cells derived from embryonic stem cells of the present invention for a long period, the medium is preferably stored frozen at -10°C - -80°C. When the conditioned medium of astrocyte-like cells derived from embryonic stem cells of the present invention is stored frozen, repeated freeze-thaw should be avoided so as to prevent decreasing in activities of various proteinous factors which are responsible for the function of said medium. In addition, the medium can also preferably be stored in refrigerator at 4-8°C.

The conditioned medium of astrocyte-like cells derived from embryonic stem cells of the present invention can be provided by itself or as a kit including the medium and, for example, suitable factors or reagent for culturing embryonic stem cells, neural cells or cells of each animal species, suitable factors or reagents for inducing cell differentiation, a culture vessel coated with cell adhesion molecule, a culture vessel suitable for suspension culture, or the like.

A medium with the similar effects to those of the conditioned medium of astrocyte-like cells derived from embryonic stem cells of the present invention may be prepared by using various functionally active substances (hereinafter called as "functional component(s)") identified and isolated from the conditioned medium of astrocyte-like cells derived from embryonic stem cells of the present invention so that the medium contain effective amounts of the functional components. Therefore, one embodiment of present invention includes preparing a functional medium having the similar ability to culture neural cells and induce differentiation of embryonic stem cells to neural cells compared to the conditioned medium of astrocyte-like cells derived from embryonic stem cells of the present invention by mixing the functional components. In the method, the functional components may be supplied individually and mixed individually before use. The present invention includes a medium prepared by mixing said functional components. In addition, said functional components include a component having an ability to facilitate the culture of neural cells and the differentiation of embryonic stem cells into neural cells by itself. Such sole use of a functional component is also included in the scope of the present invention. Said functional component can be provided by itself or in combination of at least 2 kinds of them as the reagents which enables to culture and/or maintain neural cells and/or to induce differentiation of embryonic stem cells to neural cells.

In another embodiment, the present invention provides a method for culturing neurons, characterized in that the conditioned medium of astrocyte-like cells derived from embryonic stem cells is employed for the culture. Specifically, neurons are cultured in the presence of the conditioned medium of astrocyte-like cells derived from embryonic stem cells. The method for culturing neurons of the present invention can maintain a stable and healthy neural culture containing substantially no component other than those derived from the targeted animal.

In the culture method of the present invention, animal species used as a source of neurons may include, but are not limited to, mammals, such as mouse, rat, mink, hamster, pig, dog, sheep, goat, monkey and human.

According to the culture method of the present invention, in case the resulting neuronal cell culture is used for the cell transplantation therapy, the conditioned medium of astrocyte-like cells derived from embryonic stem cells is preferably prepared from the embryonic stem cells derived from the same species as the animal to be treated in view of good compatibility.

The culture method of the present invention can be applied to any methods for culturing neurons.

Neurons may be cultured in the presence of the conditioned medium of astrocyte-like cells derived from embryonic stem cells of the present invention under a suitable conditions for the culture. The culture conditions other than using the conditioned medium of astrocyte-like cells derived from embryonic stem cells are not specifically limited and the culture may be conducted by using a culture supernatant of astrocyte like cells derived from embryonic stem cells in the DMEM:F-12 medium containing 1× N2 supplement [5µg/ml insulin, 100µg/ml transferrin, 63ng/ml progesterone, 16.11µg/ml putrescine and 2ng/ml selenite] in the presence of the conditioned medium of astrocytes derived from embryonic stem cells, or the conditioned medium of astrocyte-like cells derived from embryonic stem cells of the present invention supplemented with 1× N2 supplement; at about 37°C (e.g. 37±0.2°C) and about 5% CO₂ (e.g. 4.8-5.2%); in a culture vessel coated with poly-L-lysine, laminin, or the like.

In another aspect, the present invention provides a method for differentiating embryonic stem cells into neural cells, characterized in that the conditioned medium of astrocyte-like cells derived from embryonic stem cells prepared by the method of the present invention is employed as a medium. The differentiation of embryonic stem cells to neural cells is induced, for example, in the presence of the conditioned medium of astrocyte-like cells derived from embryonic stem cells prepared by the method of the present invention. According to the method for differentiating the present invention, neural cells containing no component derived from an animal species other than the targeted animal can be prepared. That is, upon preparing the conditioned medium of astrocyte-like cells derived from embryonic stem cells of the present invention, the embryonic stem cells derived from same animal species as the neural cells to be cultured are used. Therefore, the neural cells prepared by the method for inducing differentiation of the present invention can preferably be used for the cell transplantation therapy. In addition, the method for inducing differentiation of the present invention has an advantage in preparing human neural cells containing substantially no non-human component which are suitable for the clinical application to a human patient such as the neural cell transplantation treatment.

The embryonic stem cells used in the method for inducing differentiation of the present invention can conveniently be selected depending on the purpose of use of the resulting neural cells. The animal species used as a source of embryonic stem cells which are used in the method for inducing differentiation of the present invention may include, but are not limited to, for example mammals, such as mouse, rat, mink, hamster, pig, dog, sheep; goat, monkey, human or the like.

The method for inducing differentiation of the present invention can be conducted according to the above-described step 1') of the step (A) of the above mentioned method for preparing the conditioned medium.

The prepared neural stem cells can be evaluated by testing the ability to be differentiated into neural cells (neurons or glia cells), the expression of markers of said neural stem cells or the like.

According to the present method, the induction of differentiation from embryonic stem cells into neurons can be achieved as follows; conducting suspension culture of a colony of embryonic stem cells in the conditioned medium of astrocyte-like cells derived from embryonic stem cells to give a cell sphere (NSS) which comprises a layer of undifferentiated embryonic stem cells in the core (central) and a layer of a number of neural stem cells on the surface; conducting adherent culture of said prepared NSS in the presence of the conditioned medium of astrocyte-like cells derived from embryonic stem cells of the present invention; conducting the adherent culture of said neural stem cells in the presence of the conditioned medium of astrocyte-like cells derived from embryonic stem cells of the present invention. The prepared neurons may be identified by the expression of neuronal markers such as neurofilament, tyrosine hydroxylase, glutamic acid decarboxylase and choline acetyltransferase. In the method, a medium such as the conditioned medium of astrocyte-like cells derived from embryonic stem cells of the present invention may be used and said conditioned medium may be mixed with any one of mediums such as DMEM:F-12, DMEM, F-12, MEM, Neurobasal^{™} and the like, or supplemented with further additives. The culture may be conducted under an atmosphere of about 5% CO2 such as 4.8-5.2%, 100% humidity at about 37°C, such as 37±0.2°C. The period for the culture may be determined depending on the species of animals from which the embryonic stem cells are derived, and it may desirably be 1-7 days in case of a mouse and 1-14 days in case of a monkey.

Whether the prepared cells are neurons or not can be confirmed by examining, for example, morphological features of the cells including cell body, dendrite, axon, growth cone or the like. In addition, prepared neurons can be evaluated by examining the expression of the markers such as neurofilament, tyrosine hydroxylase, glutamic acid decarboxylase, choline acetyltransferase or the like, or the coding gene thereof.

The induction of differentiation from embryonic stem cells into glia cells according to the method of the present invention can be achieved by culturing the above mentioned NSS or neural stem cells in adherent culture. The mediums which can be used for this method include, but are not limited to, DMEM:F-12 + N2 supplement (N2 medium) and the like. The period for the culture can be determined depending on the types of embryonic stem cells, and it may desirably be 1-7 days in case of mouse embryonic stem cells and 1-4 days in case of monkey embryonic stem cells. In addition, the prepared glia cells can be identified by examining the morphological features or the expression of the markers for glia cells such as glial fibrillary acidic protein (GFAP) for astrocytes.

The neural cells prepared by the method for inducing differentiation of the present invention can be prepared in the absence of any components derived from animals other than the targeted animal, for example, in the absence of non-human component. Therefore, said neural cells containing substantially no component derived from an animal other than the targeted animal can effectively be used for cell transplantation therapy. In addition, according to the method for inducing differentiation of the present invention, the neural cells are prepared by using the conditioned medium of astrocyte-like cells derived from embryonic stem cells. By inducing the differentiation of an animal's embryonic stem cells using the conditioned medium of astrocyte-like cells which is derived from embryonic stem cells of said animal, such as human, neural cells having high compatibility with the living body of the animal and containing substantially no component derived from animals other than the targeted animal can be provided. Therefore, the neural cells of the present invention can be utilized for an assay system for drug discovery which is useful for the targeted animal. The neural cells of the present invention can be applied to the regenerative treatment of the nerve system targeted for the disease or the condition caused by neurodegeneration or nerve injury such as neurodegerative disease including Parkinson's disease, Alzheimer's disease, amyotrophic lateral sclerosis; brain ischemia, demyelinating disease, head injury, spinal cord injury, stroke or the like; in the regenerative treatment, the neural cells of the present invention are introduced in an area of neurodegeneration or or nerve injury is observed. Such neural cells are also included in the present invention.

### EXAMPLE

Reference is now made to the following examples, which illustrate the present invention specifically, but the following examples never limit the present invention. In the following examples, "%" refers to weight % unless otherwise stated, however, "%" regarding CO₂ refers to volume %.

### EXAMPLE 1

The mouse embryonic stem cell line, 129SV cells were cultured in the usual manner and 3 days after the cell seeding, a whole colony of cells were physically picked up using a capillary. The resulting colony were cultured in a suspension at 37°C, 5% CO₂ for 4 days using a culture medium for inducing neural differentiation (which was used as an alternative for the conditioned medium of primary astrocyte cells; Product No.MB-X9501, Sumitomo Bakelite Co., Ltd.), and thereby Neural stem sphere (NSS) was prepared, which was composed of 3 layers: a layer of undifferentiated embryonic stem cells in the center; a superficial layer of neural stem cells: and an interlayer between both layers.

Then, said NSS was plated on a dish coated with poly-L-lysine/laminin and cultured using Neurobasal^{™} medium [catalog No:21103-049, Invitrogen Corporation] containing 1×B27 supplement [catalog No:17504-044, Invitrogen Corporation; adjusted from commercially available X50 product] at 37°C, 5% CO₂ with adding basic fibroblast growth factor (bFGF) every day at the final concentration of 20ng/ml to proliferate the neural stem cells. The above mentioned dish coated with poly-L-lysine/laminin was prepared by coating commercially available poly-L-lysine coated dish [IWAKI, Asahi Techno Glass Corporation] with 10µg/ml laminin [catalog No:L0001, Asahi Techno Glass Corporation] for 3 hours.

After 7 days culture, undifferentiated cells in the center of the proliferating cells were removed physically using a capillary pipette so as to increase the abundance ratio of the migrated and proliferated neural stem cells, and then, the culture was incubated at 37°C, 5% CO₂ for further 7 days to proliferate the neural stem cells.

After that, the proliferating cells were dissociated using 0.05 % by weight of trypsin-EDTA after discarding the medium and washing the cells with phosphate buffered saline without containing Ca²⁺ and Mg²⁺ so as to induce selective differentiation of the neural stem cells to astrocyte-like cells. Said dissociated cells were subcultured in DMEM:F-12 medium containing N2 supplement [5µg/ml insulin, 100µg/ml transferrin, 6.3ng/ml progesterone, 16.11µg/ml putrescine, 5.2ng/ml selenite, Catalog No:17502-048, Invitrogen Corporation].

After 3 days culture, the supernatant were removed from the resulting culture, filtered by 22 nm-filter [trade name: DISMIC-25CS, Advantech Co., Ltd.], to provide the conditioned medium of astrocyte-like cells derived from embryonic stem cells. N2 supplement at the final concentration of 1% [×1 N2 supplement was adjusted from x100 product] was added to the prepared conditioned medium of astrocyte-like cells to make up for the nutrition.

### EXAMPLE 2

In order to prepare neurons for examining the ability to culture neurons, frozen mouse fetal cortex [trade name of neuronal culture system [made by Sumitomo Bakelite]:SHINKEISAIBOU CX(M) [Catalog No:MB-X0305]] was thawed at room temperature, and dispersed in the cell dispersion liquid [Catalog No:MB-X9901] included in the neuron culture system [made by Sumitomo Bakelite] to provide a suspension containing primary neurons derived from mouse fetal cortex.

Then, said suspension containing primary neurons derived from mouse fetal cortex were centrifuged at 300rpm for 1 min using himac CF702 [made by Hitachi] to wash the primary neurons. Then, the primary neurons derived from mouse fetal cortex were suspended in the conditioned medium of astrocyte-like cells derived from mouse embryonic stem cells prepared according to the above Example 1 at 1X10⁵ cells/ml, and plated the cells in 0.5 ml/well on the poly-L-lysine coated 24 well plate [made by Sumitomo Bakelite Corporation] to be cultured at 37 °C, 5% CO₂. Half of the medium was replaced with fresh medium every 2 days. For the control, the primary neurons derived from mouse fetal cortex was cultured in N2 medium in a similar way for using the conditioned medium of astrocyte-like cells derived from mouse embryonic stem cells. Figure 1 illustrates morphology of cells after 2 days culture. In Figure 1, Panels A and B indicate the case of using N2 medium, and Panels C and D indicate the case of using the conditioned medium of astrocyte-like cells derived from mouse embryonic stem cells.

As shown in Panels C and D of Figure 1, very excellent adhesion and elongation of neurites were observed in case of using the conditioned medium of astrocyte-like cells derived from mouse embryonic stem cells. On the other hand, as shown in Panels A and B, insufficient cell adhesion and little elongation of neurites from the adherent cells were observed in case of using N2 medium.

On day 4 of the culture, the medium was removed from the culture. 4 % by weight of paraformaldehyde was added to the cultured cells and the cells were incubated for 5 minutes at room temperature. Then, 1ml of 0.1% Triton^{™}-X was added to the resulting mixture and the mixture was incubated for further 5 minutes. 1% by weight of Normal Goat Serum was added to the resulting mixture and the mixture was incubated for more 30 minutes at room temperature to allow blocking.

Anti-GFAP antibody [catalog No:AB5804, CHEMICON] and anti-MAP2 antibody [catalog No: 442695, CALBIOCHEM], diluted with 1% by weight of Normal Goat Serum were added to the blocked cells and the mixture was incubated at 4°C overnight to allow the reaction. Then, the cells were washed with phosphate buffered saline without Ca²⁺ and Mg²⁺, and the second antibody [trade name: Alexa Fluor 488; Molecular Probe] was added to the cells and incubated for 30 minutes at room temperature to allow the reaction. Then, the expression of glial fibrillary acidic protein (GFAP) and MAP2 in the cells were detected respectively based on the fluorescence of said second antibody. The results of the immunostaining are presented in Figure 2. In the figure, Panel A shows the case of using N2 medium and Panel B shows the case of using the conditioned medium of astrocyte-like cells derived from mouse embryonic stem cells.

As shown in Figure 2, in case of using the conditioned medium of astrocyte-like cells derived from embryonic stem cells, very high expression of MAP2 was observed, while little expression of GFAP was observed in the cells. The result indicates that only neuronal cells are proliferated well by using the conditioned medium of astrocyte-like cells derived from embryonic stem cells. On the other hand, as shown in Panel A of Figure 2, the culture using N2 medium provided neither GFAP-positive cells or MAP2-positive cells.

In addition, on Day 2 of the culture, the conditioned medium in some cultures prepared by the above Example 1 was replaced with Neurobasal^{™} medium containing 1xB-27 supplement [Invitrogen Corporation] and the cells were further incubated at 37 °C, 5% CO₂ for 10 days to evaluate effect of the conditioned medium of astrocyte-like cells derived from embryonic stem cells on long term culture.

The result indicated that cells cultured using N2 medium from the beginning of the culture were died on Day 5.

Day 8 of the cell cultures using conditioned medium of astrocyte-like cells derived from embryonic stem cells and Neurobasal^{™} medium containing B-27 supplement are shown in Figure 3 and Day 10 of them are shown in Figure 4. In each Figure, Panels A and B show the cells cultured in Neurobasal^{™} medium containing B-27 supplement and Panels C and D show the cells cultured in the conditioned medium of astrocyte-like cells derived from embryonic stem cells.

As shown in Panels C and D in each of Figures 3 and 4, neural cells cultured using the conditioned medium of astrocyte-like cells derived from embryonic stem cells maintained dense networks between neurons even on Day 10.

On the other hand, neural cells cultured using Neurobasal^{™} medium containing B-27 supplement began to die from Day 7, and as shown in Panels A and B of Figure 3, most of the cells were dead on Day 8, and as shown in Panels A and B of Figure 4, all the cells were dead on Day 10.

These results indicated that by using the conditioned medium of astrocyte-like cells derived from embryonic stem cells, neurons such as primary neurons can be maintained stably in vitro for a long time. In the past, it has been difficult to maintain neurons in a healthy condition for a long time.

### EXAMPLE 3

The ability of the conditioned medium of astrocyte-like cells derived from embryonic stem cells prepared by the above Example to induce differentiation from mouse embryonic stem cells to neurons was examined as follows.

The mouse embryonic stem cell line, 129SV cells were cultured for 3 days using a conventional method so that large and grown-up colonies were formed. Colonies of the cells were picked up using a capillary pipette, and cultured in suspension at 37°C, 5% CO₂ for 4 days, in the conditioned medium of astrocyte-like cells derived from embryonic stem cells prepared by the above Example 1 and in N2 medium as control, respectively.

After that, the resulting colonies were cultured on poly-L-lysine coated dish (Asahi Techno Glass Corporation] at 37°C, 5% CO₂ for 7 days, using respective medium so that the colonies were differentiated into neural cells.

The expression of Class III β tubulin in the resulting cell populations was analyzed by immunostaining with anti-βIII tubulin (TuJ) antibody to examine the extent of the differentiation from mouse embryonic stem cells to neurons (i.e. the number of neural differentiated colonies).
The results are shown in Table 1. In the table, ESACM indicates the conditioned medium of astrocyte-like cells derived from embryonic stem cells and N2 indicates N2 medium. In the table, the numerical value in parentheses indicates a ratio of the number of positive cells which is calculated according to the formula : (total number of positive colonies/ number of picked up colonies) X100 (%).

**[Table 1]**

| medium | number of picked up colonies | number of seeded cells | number of colonies expressing βIII tubulin | | | total number of positive colonies |
|---|---|---|---|---|---|---|
| | | | ++ | + | +- | |
| ESACM | 105 | 82 (78) | 25 (24) | 20 (19) | 9 (9) | 54 (51) |
| N2 | 105 | 46 (44) | 3 (3) | 4 (4) | 11 (10) | 18 (17) |

As shown in Table 1, a ratio of positive colonies was 51% in case of using the conditioned medium of astrocyte-like cells derived from embryonic stem cells prepared according to Example 1. It is clearly indicated that said conditioned medium of astrocyte-like cells has an enough ability to induce differentiation from embryonic stem cells to neural cells.

In addition, the proliferating ability of the neural stem cells differentiated by using the conditioned medium of astrocyte-like cells derived from embryonic stem cells prepared according to the above Example 1, was determined by culturing the cells at 37 °C, 5% CO₂ in Neurobasal^{™} medium [Invitrogen Corporation] and observing the resulting cells under microscope [Nikon Corporation, trade name: ECLIPSE TE2000-U].
The results showed that the neural stem cells differentiated by using the conditioned medium of astrocyte-like cells derived from embryonic stem cells according to Example 1 had a good proliferating ability.

In addition, after culturing said proliferated neural stem cells for 7 days using the conditioned medium of astrocyte-like cells derived from embryonic stem cells according to Example 1, the cells were immunostained with MAP2 and microscopically observed to examine the extent of differentiation of the cells into neurons.
The results indicated that said proliferated cells were differentiated into cells with neuronal morphology and most of the cells were MAP2-positive. Therefore, it is suggested that the neural stem cells obtained by using the conditioned medium of astrocyte-like cells derived from embryonic stem cells prepared according to Example 1 had the proliferating and differentiation potential.

The monkey embryonic stem cell line, CMK6 cells were differentiated to neural cells using the conditioned medium of astrocyte-like cells derived from the embryonic stem cells under a condition suitable for monkey's cells in a similar way as described in Example 2. The results are shown in Table 2. In the table, ESACM indicates the conditioned medium of astrocyte-like cells derived from embryonic stem cells and N2 indicates N2 medium.

**[Table 2]**

| medium | number of picked up colonies | number of seeded cells | number of colonies expressing βIII tubulin | | | total number of positive colonies |
|---|---|---|---|---|---|---|
| | | | ++ | + | +- | |
| ESACM | 40 | 27 (68) | 17 (43) | 5 (13) | 4 (10) | 26 (65) |
| N2 | 40 | 16 (40) | 6 (15) | 5 (13) | 3 (8) | 14 (35) |

As shown in Table 2, the results clearly indicated that the conditioned medium of astrocyte-like cells derived from embryonic stem cells had an enough ability to induce differentiation of monkey embryonic stem cells to neural cells. In addition, the proliferation potency of the obtained cells was examined in the same manner as mouse embryonic stem cells and confirmed the prepared monkey neural stem cells had a good proliferating potency.

### EXAMPLE 4

The cynomolgus monkey ES cell line, CMK6 cells were cultured using a conventional method, and on Day 3 after the cell seeding, colonies of the cells were picked up physically using a capillary. The resulting colonies were cultured using the medium for neuronal culture (Sumitomo Bakelite: Catalog No:MB-X9501, used as alternative to the conditioned medium of primary astrocyte culture) containing 20ng/ml bFGF at 37°C, 5% CO₂ for 10 days in suspension to prepare Neural stem sphere (NSS). During the 10 days suspension culture, basic fibroblast growth factor (bFGF) was added to said suspension culture in a final concentration of 20ng/ml everyday.

Then, said NSS was plated on poly-L-lysine/laminin coated dish and cultured using Neurobasal^{™} medium [Catalog No:21103-049, made by Invitrogen Corporation] containing 1xB27 supplement [catalog No: 17504-044, Invitrogen Corporation, adjusted from commercially available ×50 product] and adherent-cultured at 37 °C, 5% CO₂ for 7 days with adding bFGF and epidermal growth factor (EGF) at the final concentration of 20ng/ml everyday to proliferate the neural stem cells.

The above poly-L-lysine/laminin coated dish was prepared by coating poly-L-lysine coated dish [IWAKI, Asahi Techno Glass Corporation] with 10µg/ml laminin [Catalog No:L0001, Asahi Techno Glass Corporation] for 3 hours.

After 7 days culture, the central part of the proliferating cells was physically transferred on the poly-L-lysine/laminin coated dish using a capillary, and then, the culture of the cells was continued using Dulbecco's Modified Eagle Medium (DMEM) [Catalog No:11960-069, Invitrogen Corporation] containing ×1 G-5 supplement [Catalog No:17503-012, Invitrogen Corporation; ×100 product] (hereinafter referred as G-5 medium). The culture at 37°C, 5% CO₂ was continued for 7 days. During the above culturing period, bFGF and EGF were added at each final concentration of 20ng/ml everyday.

On Day 7 of the culture, the central part of the proliferating cells was physically removed using a capillary and the remaining cells were cultured for further 7 days.
Then, G-5 medium was discarded, and the cells were washed phosphate buffered saline without Ca²⁺ and Mg²⁺, and then, said cells were dissociated with 0.05% by weight of trypsin/EDTA. The resulting dissociated cells (the proliferated neural stem cells) were cultured on the poly-L-lysine/laminin coated dish using G-5 medium again for 10 days to induce differentiation into astrocyte-like cells.

Figure 5 illustrates an immunostaining of the culture derived from monkey embryonic stem cells prepared in the above way with anti-GFAP antibody [Catalog No: AB5804, CHEMICON Corporation] and anti-MAP2 antibody [Catalog No:442695, CALBIOCHEM Corporation] in a conventional manner. In the figure, Panel A indicates the expression of GFAP, Panel B indicates the expression of MAP2, Panel C indicates the merged image of Panels A and B.

Most of the cells in the culture vessel were astrocyte-like cells (GFAP positive), and little neurons which were MAP2 positive were existed in the vessel. Thus, the result indicated that highly pure and homogeneous monkey astrocyte-like cells were prepared.

Then, the medium was replaced with N2 medium (i.e. DMEM/F-12 medium supplemented with ×1 N2 supplement) and the culture was continued. 2-4 days later, the supernatant was recovered from the culture and said supernatant was filtrated with a 22nm-filter [trade name:DISMIC-25CS, ADVANTEC] to obtain the conditioned medium of astrocyte-like cells derived from monkey embryonic stem cells.
Then, said conditioned medium of astrocyte-like cells derived from monkey embryonic stem cells was supplemented with N2 supplement [Catalog No:17502-048, Invitrogen Corporation; ×100 product] or B-27 supplement [Catalog No:17504-044, Invitrogen Corporation; ×50 product] in an amount to give ×1 concentration, respectively, and the ability of the medium to induce differentiation of the embryonic stem cells into neurons and to culture neurons stably for a long time were examined.

### EXAMPLE 5

The ability of conditioned medium of astrocyte-like cells derived from monkey embryonic stem cells prepared according to Example 4 to induce differentiation of CMK6-G4 cell line (a cell line stably expressing hrGFP gene) to neurons was examined.
CMK6-G4 cell line was cultured using a conventional method, and on Day 3 after cell seeding, colonies of the cells were physically picked up using a capillary. The obtained colonies were cultured at 37°C, 5% CO₂ for 10 days in suspension using the conditioned medium of astrocyte-like cells derived from monkey embryonic stem cells (prepared according to Example 4) containing 20ng/ml bFGF to give Neural stem spheres (NSS). During the 10 days suspension culture, bFGF was added to the suspension culture at the concentration of 20ng/ml everyday.

Then, the obtained NSS was plated on the poly-L-lysine/laminin coated dish and cultured using the same conditioned medium of astrocyte-like cells derived from monkey embryonic stem cells, and adhesive-cultured at 37°C, 5% CO₂ for 14 days to induce differentiation of the NSS into neural cells. A number of neurites were observed under a microscope.

To confirm the differentiation of monkey embryonic stem cells to neurons, the expressions of class III β tubulin and MAP2 in the prepared population were evaluated by immunostaining the cells with anti-βIII tubulin (Tuj) antibody [Catalog No: MAB1637, CHEMICON Corporation] and anti-MAP2 antibody [Catalog No:442695, CALBIOCHEM Corporation] respectively in a conventional manner. The results are shown in Figure 6.

As shown in Figure 6, it can be recognized that a number of cells (neurons) expressing βIII tubulin or MAP2 were induced when the conditioned medium of astrocyte-like cells derived from monkey embryonic stem cells was used.
These results indicate that the conditioned medium of astrocyte-like cells derived from monkey embryonic stem cells had an ability to induce differentiation of monkey embryonic stem cells to neural stem cells and also had an ability to induce differentiation of the neural stem cells to neurons efficiently.

On the other hand, in the control groups where N2 medium (i.e. a basal medium used for preparing the conditioned medium) and B27 medium (i.e. Neurobasal^{™} medium containing B-27 supplement) were used for the culture, only little neurons, which might be due to the spontaneous differentiation, were observed and no substantial induction of differentiation to neurons was recognized (data not shown).

### EXAMPLE 6

A human ES cell line, SA181 cells were cultured in a conventional manner, and on Day 3 after the cell seeding, colonies of the cells were physically picked up using a capillary. The obtained colonies were cultured at 37°C, 5% CO₂ for 12 days in suspension using a medium for neuronal culture [Sumitomo Bakelite Co., Ltd.: Catalog No:MB-X9501, used as an alternative to the conditioned medium of primary astrocyte] containing 20ng/ml bFGF to prepare Neural stem sphere (NSS). During the suspension culture, bFGF was added to the suspension culture at the concentration of 20ng/ml everyday.

Then, said NSS was plated on the poly-L-lysine/laminin coated dish and cultured using Neurobasal^{™} medium [Catalog No:21103-049, Invitrogen Corporation] containing 1×B-27 supplement [Catalog No:17504-044, Invitrogen Corporation, adjusted from commercially available ×50 product], and adherent-cultured at 37°C, 5% CO₂ for 7 days with addition of bFGF and EGF at a final concentration of 20ng/ml respectively to induce proliferation of the neural stem cells.

In addition, the above poly-L-lysine/laminin coated dish was prepared by coating commercially available poly-L-lysine coated dish [IWAKI, Asahi Techno Glass Corporation] with 10µg/ml laminin [Catalog No:L0001, Asahi Techno Glass Corporation] for 3 hours.

After culturing for 7 days, the central part of the proliferating cells was physically transferred on the poly-L-lysine/laminin coated dish using a capillary, and then, the culture of the cells using Dulbecco's Modified Eagle Medium (DMEM) [Catalog No:11960-069, Invitrogen Corporation] supplemented with ×1 G-5 supplement [Catalog No:17503-012, Invitrogen Corporation; ×100 product](hereinafter called as G-5 medium) was continued. The culture of the cells were continued at 37°C, 5% CO₂ for 7 days. During the culture, bFGF and EGF were added at each final concentration of 20ng/ml everyday.

On Day 7 of the culture, the central part of the proliferating cells was physically removed using a capillary and the culture of the remaining cells were continued for further 7 days.
Then, G-5 medium was discarded and the cells were washed with phosphate buffered saline without Ca²⁺ and Mg²⁺, and then, said cells were dissociated with 0.05% by weight of trypsin/EDTA. The dissociated cells (the proliferated neural stem cells) were cultured on the poly-L-lysine/laminin coated dish using G-5 medium again for 14 days to differentiate into astrocyte-like cells.

Figure 7 illustrates the results of immunostaining of thus obtained culture cells prepared from human embryonic stem cells with anti-GFAP antibody and anti-MAP2 antibody.
As shown in Figure 7, it can be recognized that most of the cells in the culture vessel were astrocyte-like cells (GFAP positive), and little neurons which were MAP2 positive were existed in the vessel. Thus, the result indicated that highly pure and homogeneous astrocyte-like cells were prepared.

Then, the medium was replaced with N2 medium (i.e. DMEM/F-12 medium supplemented with ×1 N2 supplement) and the cells were continued to culture. 2-4 days later, the supernatant was recovered from the culture and filtered by a 22nm-filter [trade name:DISMIC-25CS, ADVANTEC] to obtain the conditioned medium of astrocyte-like cells derived from human embryonic stem cells.
Then, said conditioned medium of astrocyte-like cells derived from human embryonic stem cells was supplemented with ×1 N2 supplement [Catalog No:17502-048, Invitrogen Corporation; ×100 product] or ×1 B-27 supplement [Catalog No:17504-044, Invitrogen Corporation; ×50 product]. EXAMPLE 7

The ability of the conditioned medium of astrocyte-like cells derived from human embryonic stem cells prepared according to Example 6 to induce differentiation of SA181hrG2 cell line (a cell line stably expressing hrGFP gene; which is designed to generates green fluorescence to facilitate identification of the cells) to neurons was evaluated.
SA181hrG2 cell line was cultured in a conventional manner, and on Day 3 after cell seeding, colonies of the cells were physically picked up using a capillary. The obtained colonies were cultured at 37 °C, 5% CO₂ for 12 days in suspension using the conditioned medium of astrocyte-like cells derived from human embryonic stem cells (prepared according to Example 6) containing 20ng/ml bFGF to prepare Neural stem spheres (NSS). During the 12 days suspension culture, bFGF was added to the suspension culture at the concentration of 20ng/ml everyday.

Then, said NSS was plated on the poly-L-lysine/laminin coated dish and cultured using the same conditioned medium of astrocyte-like cells derived from human embryonic stem cells as above, and adherent-cultured at 37°C, 5% CO₂ for 14 days to induce differentiation of the NSS into neural cells. In the obtained culture, a number of neurites were observed under a microscope.

To confirm whether the human embryonic stem cells were differentiated into neurons or not, the expressions of class III β tubulin and MAP2 in the prepared population were evaluated by immunostaining the resulting cells with anti-βIII tubulin (Tuj) antibody [Catalog No: MAB1637, CHEMICON Corporation] and anti-MAP2 antibody [Catalog No:442695, CALBIOCHEM Corporation] respectively in a conventional manner. The results are shown in Figure 8.

As shown in Figure 8, it can be recognized that a number of neurons (i.e. cells expressing βIII tubulin or MAP2) were induced when the conditioned medium of astrocyte-like cells derived from human embryonic stem cells was used.
These results indicate that the conditioned medium of astrocyte-like cells derived from human embryonic stem cells had an ability to induce differentiation of human embryonic stem cells to neural stem cells and also had an ability to induce differentiation of the neural stem cells to neurons efficiently.

On the other hand, in the control groups where N2 medium (i.e. a basal medium used for preparing the conditioned medium) and B27 medium (i.e. Neurobasal^{™} medium containing B-27 supplement) were used for the culture, only little neurons, which might be due to the spontaneous differentiation, were observed and no substantial induction of differentiation to neurons was recognized.

In addition, when the conditioned medium of astrocyte-like cells derived from human embryonic stem cells prepared according to Example 6 was used for culturing neuronal cultures derved from various sources including primary-cultured neurons derived from mouse fetal cortex [Catalog No:MB-X0305: SHINKEISAIBOU CX(M), Sumitomo Bakelite Co., Ltd.], human normal neural precursor cells [Product Code:PT-2599, Cambrex Corporation], and neurons derived from human embryonic stem cells, the neuronal cells were maintained and a number of neurites were observed under a microscope. The above results are similar to those as shown in Example 2.

Figure 9 illustrates the results regarding human neural precursor cells. When N2 medium (i.e. the basal medium for preparing the conditioned medium) was used for the culture, such areas as found in Panel A are sparsely observed in the culture vessel. On the other hand, when hES-ACM was used, a number of neurons were found over the whole area of the culture vessel, and thus it can be understood that the hES-ACM had an excellent ability to culture neurons.

### EXAMPLE 8

The conditioned medium was prepared by the similar method as above using mouse astrocyte cell line, KT-5 cells (GFAP positive; resource No. of Human Science Research Resource Bank: IFO50161). Then, the abilities of said conditioned medium to induce differentiation of ES cells to neurons and to culture neurons stably for a long time were examined.

Mouse KT-5 cells were subcultured using Nutrient Mixture F-12 HAM medium (Catalog No. N8641; made by SIGMA Co.) containing 10% fetal calf serum in a conventional manner. When the culture reached 70% confluent, the medium was replaced with DMEM/F-12 medium containing ×1 N2 supplement. After further 2 days culture, the supernatant was recovered from the culture and filtrated with 22nm-filter[trade name: DISMIC-25CS, ADVANTEC] to provide the conditioned medium of mouse KT-5 cells.

Then, said conditioned medium was supplemented with ×1 N2 supplement [Catalog No:17502-048, Invitrogen Corporation; ×100 product] or ×1 B-27 supplement [Catalog No:17504-044, Invitrogen Corporation; ×50 product], and the ability to induce differentiation of mouse embryonic stem cells to neural cells and the ability to culture neurons in vitro stably for a long time were examined.
However, it was found that the conditioned medium of KT-5 cells could neither culture neurons stably for a long time nor induce differentiation of the embryonic stem cells to neural cells efficiently.

This result gives us one example indicating that there is no guarantee that every conditioned medium of every GFAP positive cells (i.e. astrocytes) can be used to culture neurons stably for a long time or to induce differentiation of the embryonic stem cells to neural cells (i.e. the conditioned medium prepared by culturing one type of astrocytes does not always have the same ability as the conditioned medium of astrocyte-like cells derived from embryonic stem cells).

The above Examples make it clear that the conditioned medium of astrocyte-like cells derived from embryonic stem cells of the present invention is useful for maintaining a neuronal culture stably for a long time and inducing differentiation of embryonic stem cells to neural cells, and the like.

### INDUSTRIAL APPLICABILITY

According to the present invention, a large amount of conditioned medium of astrocyte-like cells can be provided stably and readily. The conditioned medium enables stable manufacturing of neural cells, industrial manufacturing of neural cells; culturing neurons stably for a long time; inducing differentiation of embryonic stem cells to neural cells efficiently; supplying neural cells prepared by inducing differentiation of embryonic stem cells to neural cells in an industrial scale, etc. Thus, the present invention makes it possible to readily supply a large amount of neural cells with high quality. In addition, because the present invention can maintain or prepare neural cells under the condition where any cell components derived from animals other than the targeted animal are substantially absent, the present invention enables to supply materials suitable for the cell transplantation therapy and the like having a high compatibility with a living body.

## Claims

1. A method for preparing a conditioned medium of astrocyte-like cells derived from embryonic stem cells, **characterized in that** astrocyte-like cells, which are prepared by differentiation of embryonic stem cells, are cultured.

2. The method of Claim 1, wherein said embryonic stem cells are mammalian embryonic stem cells.

3. The method of Claim 2, wherein said mammal is a primate.

4. The method of Claim 3, wherein said primate is human.

5. The method of any one of claims 1-4, comprising the steps of:
(A) preparing astrocyte-like cells from embryonic stem cells, and
(B) culturing the astrocyte-like cells prepared by said step (A) to give culture supernatant.

6. The method of Claim 5, in step (A) of the method, the embryonic stem cells are differentiated by using an conditioned medium of astrocytes, a preliminarily prepared conditioned medium of astrocyte-like cells, or a preliminarily prepared medium which is functionally-equivalent to said conditioned medium of astrocytes or conditioned medium of astrocyte-like cells.

7. A conditioned medium of astrocyte-like cells derived from embryonic stem cells, which is prepared by the method of any one of Claims 1-6.

8. A method for culturing neurons, **characterized in that** the neurons are cultured in the presence of the conditioned medium of astrocyte-like cells derived from embryonic stem cells according to Claim 7.

9. Neurons which are cultured by the method according to Claim 8.

10. A method for inducing differentiation of embryonic stem cells into neural cells, **characterized in that** the embryonic cells are differentiated into neural cells in the presence of the conditioned medium of astrocyte-like cells derived from embryonic stem cells according to Claim 7.

11. Neural cells which are obtained by differentiating embryonic stem cells using the conditioned medium of astrocyte-like cells derived from embryonic stem cells according to Claim 7.
